# EUROPEAN PATENT SPECIFICATION

(11) **EP 0 541 699 B1**
(45) Date of publication and mention of the grant of the patent: **29.05.1996**
(21) Application number: 91915175.3
(22) Date of filing: 12.07.1991
(51) Int. Cl.: A61M 36/12, A61N 5/10

(54) **DEVICE FOR INTRODUCING A RADIOACTIVE SOURCE INTO THE BODY**
VORRICHTUNG ZUM EINFÜHREN EINER RADIOAKTIVEN QUELLE IN DEN KÖRPER
DISPOSITIF D'INTRODUCTION D'UNE SOURCE RADIOACTIVE DANS LE CORPS

(30) Priority: 13.07.1990 EP 90201902
(43) Date of publication of application: 19.05.1993
(73) Proprietor: MALLINCKRODT MEDICAL, INC., St. Louis, MO 63134 (US)
(72) Inventor: BORNEMAN, Wim, NL-1742 GN Schagen (NL)
(74) Representative: Swaters, Pieter D.
(86) International application number: US9104934
(87) International publication number: WO9200776

(56) References cited:
- US-A- 3 060 924
- US-A- 3 750 653
- US-A- 4 697 575
- US-A- 4 706 652
- US-A- 4 819 618
- US-A- 4 861 520

## Description

The invention relates to a device for introducing a source of radioactive radiation into the body for therapeutic application.

Such a device is known from US-A-3,750,653. This Patent discloses a thin-walled, narrow tube which is closed at its front end and which can be introduced into the human body, in particular into the uterus. The rear end portion stays outside the body, so that after positioning the tube, a source of radioactive material can be introduced into the tube. In certain embodiments the radioactive source is positioned in the tube by means of a rod-shaped element ("preformed elongated member") in the front portion of which the radioactive material is accommodated. In other embodiments shown in the patent the radioactive material is enclosed in a short plastic sheath which in some way reaches through the thin-walled tube to the closed front end portion.

One disadvantage of the embodiments utilizing a plastic sheath is that it is difficult to accurately position the source with radioactive material because the source cannot readily be handled. Moreover, the radiation cannot simply be terminated at will by removing the source of radiation. The source of radiation can be removed only together with the thin-walled tube, which actually restricts the application to radiation therapy in those body cavities where such a thin-walled tube can be introduced and removed without much discomfort. This considerably restricts the general applicability, because many tumors which are to be considered for a radiotherapeutic treatment are not present in or in the immediate proximity of such body cavities. Finally, plastics are generally unfit for accommodating a source of radioactive radiation for therapeutic application, because plastics are not resistant to the high doses, e.g. in the order of several Curie, of radiation required for the therapeutic application, and therefore radioactive material may be released. General applicability is also restricted in the embodiments utilizing a rod-shaped element as described in the US-A-3,750,653, because many places in the body, notably the bronchi, are not accessible for such an element. This means that, although the radioactive source can be better positioned with respect to the tumor to be treated, many tumors cannot be treated radiotherapeutically by means of such a device. The use of catheterization has expanded enormously in medicine and allows for the almost unlimited possibilities of penetrating deeply into the body of a patient. However, such possibilities are seriously restricted by the use of a rod-shaped element as described in the above-mentioned United States Patent. In particular, many places which can be reached by a catheter, for example, various internal organs, cannot be reached with the radioactive source in a rod-shaped element.

US-A-4,861,520 overcomes several of the problems noted above, and provides a device for introducing a source of radioactive radiation into the body for therapeutic application which satisfies the following conditions:
(i) it is possible to position the source of radiation accurately with respect to the tumor to be treated;
(ii) the source of radiation is safely enclosed in a metal capsule which is resistant to the radioactive radiation; and
(iii) the device is generally applicable, i.e. is also usable in the radiotherapeutic treatment of those tumors which are difficult to access, in particular, lung tumors and tumors in those internal organs which are accessible by catheterization through the bloodstream or percutaneously.

The device described in US-A-4,861,520 comprises a flexible cable which can be introduced into the body through a catheter and a capsule connected coaxially to the front end of the cable, the capsule sealingly accommodating a quantity of iridium-192 or a capacity sufficient for therapeutic application. The capsule comprises a thin-walled tubular reservoir with one end sealingly connected to the flexible cable and a second end being closed and having a rounded-off external shape.

The cable which must be flexible enough to follow the track of the catheter, is manufactured from a mechanically strong and radiation-resistant material, preferably from stainless steel. In order to achieve the desired flexibility the cable is composed of a bundle of twisted steel wires. At the end of the cable remote from the capsule, a solid end portion on which information regarding the nature of the radiation source, is provided. The capsule consists of a metal reservoir, preferably made of stainless steel, which is resistant to high doses of radioactive radiation. The one end of the reservoir is sealingly closed by a plug, the plug being connected to the flexible cable by welding. The cable and capsule are proportioned so that they can be maneuvered to the desired position through a catheter already provided in the body. This is of great importance because almost any place in the body is accessible for a catheter; such as body cavities, and other places in the body. The flexibility of the cable enables the radiation source to reach places in the body which are difficult to access, in particular the deeper-situated parts of the respiratory system, as well as other internal organs, for example, liver and kidneys, which can be reached by means of a catheter either through the bloodstream or percutaneously. The length of the capsule connected axially to the cable is of essential importance, because the capsule must be manufactured from metal and is therefore rigid.

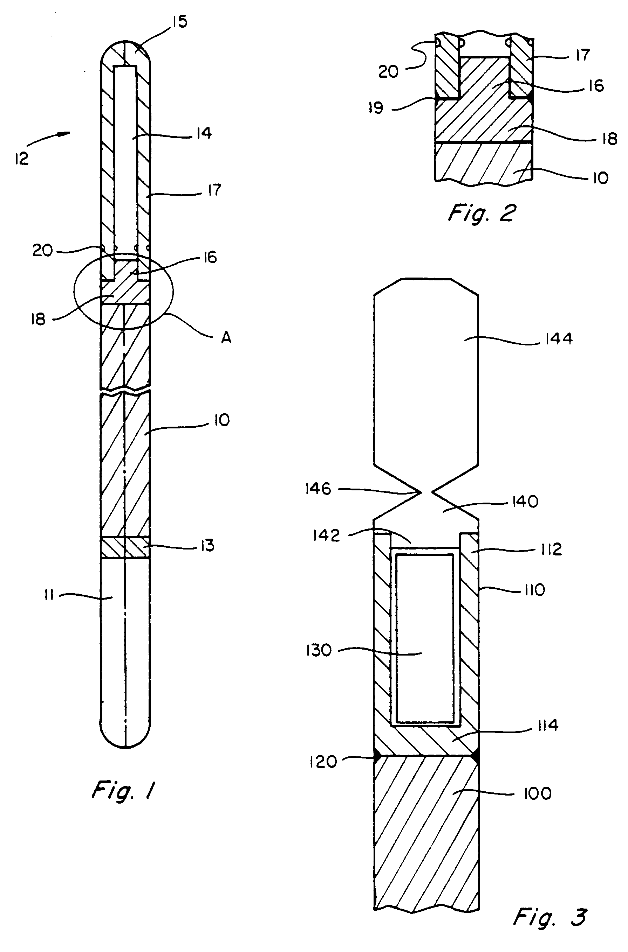

A radiation source of iridium-192 is generally used, because this isotope has very suitable radiation characteristics for therapeutic application. Further, US-A-4,861,520 uses iridium-192 in the form of pellets. A capsule having eight iridium-192 pellets, as shown in figure 3 of this patent is used to achieve the desired radiation activity of approximately 10 Curie. The length of the reservoir provided within the capsule as shown in this figure 3 embodiment is approximately 4.5 mm; which contributes considerably to the total length of 6 mm for the rigid foremost portion of the device.

It is the object of the present invention to further reduce the length of the rigid foremost portion of the device.

According to the present invention, this object can be achieved by means of a device for introducing a radioactive source into the body for therapeutic application. The device according to the present invention comprises a flexible cable which can be introduced into the body through a catheter and a capsule connected coaxially to the front end of the cable, and sealingly accommodating a quantity of iridium-192 of a capacity sufficient for therapeutic application. The capsule comprises a thin-walled tubular reservoir, one end sealingly connected to the flexible cable and a second end being closed and having a rounded-off external shape. To allow its introduction in the body through a catheter, the reservoir has an outside diameter no greater than substantially 1.1 mm. The device according to the present invention is characterized in that the capsule accommodates the radiation source in the form of a single bar instead of a plurality of pellets as described in the prior art and in that the reservoir has an external length including the sealing connection to the flexible cable of no more than substantially 4.5 mm.

It has been found that by using a radiation source in the form of a single bar a considerable reduction of the length of the reservoir can be achieved, without substantial loss of radiation activity. For example, when the eight iridium pellets of the prior art are replaced by an iridium bar according to the present invention, the length of the reservoir is reduced by approximately 1 mm, but maintains a radiation activity of approximately 10 Curie. Consequently, the total length of the rigid foremost portion of the device is reduced to about 4.5 mm at most. This reduction in reservoir length is achieved as a result of diminished play between the side wall of the reservoir and that of the bar, compared to that needed for the pellets. In loading the capsule with the radiation source, the pellets require sufficient play with regard to the inner wall of the reservoir to allow their accommodation therein, where as a bar may be introduced without substantial play. In addition, the pellets require a certain amount of mutual space between them after insertion into the reservoir, which space is eliminated when using a radiation source in the form of a bar. Also, a radiation source in the form of a bar may be accommodated in a narrow cavity more conveniently so that the manufacturing of the device according to the present invention is simplified.

The use of iridium-192 in the form of pellets as a source of radiation is common practice. Further, it is generally known in the prior art, that during manufacture by irradiation of iridium-191 in a nuclear reactor, the iridium-191 is only converted at the outside of the targets, leaving the cores unconverted. This means, that the radiation sources obtained consist of a non-radioactive core surrounded by a layer of radioactive iridium-192. In addition, the eight iridium-192 pellets normally used to reach an radiation activity of approximately 10 Curie have a surface area that is approximately 100% grater than that of a single bar of iridium-192 having the same composite length. Therefore, it was expected that such a bar or iridium-192 would have a considerably reduced radiation activity, e.g. approximately 7.5 Curie. Consequently, in the prior art, iridium-192 in the form of pellets has apparently always been used as the radiation source. Surprisingly, however, it has now been discovered that the radiation activity of a bar of iridium-192 is substantially equal to that of a plurality of pellets with the same composite length.

In one embodiment according to the present invention, the device includes a reservoir having an open end and an integrally formed closed end, the open end being hermetically sealed by means of a plug which includes an elongated portion for insertion into the reservoir. Such a construction is described in US-A-4,861,520. The back end of the flange of the plug is connected to the flexible cable by welding. In order to ensure a hermetic seal of the reservoir, the outer edge of the flange of the plug is circumferentially sealingly connected to the rear edge of the wall of the reservoir, also by welding. The plug disclosed in the above U.S. patent has a total length of approximately 1 mm, including the elongated portion which is inserted into the reservoir. In order to connect the plug to the flexible cable, the front end of the cable must be rigidly fused or welded to enable the plug welding, thus adding to the overall length of the rigid portion of the device. It has now been discovered according to a further aspect of the present invention that the total length of the rigid portion of the device can be reduced by forming the plug as an integral part of the cable. In particular, if the plug is an integral part of the cable, a separate welded portion of the cable is no longer necessary to enable welding of the plug thereto, and the rigid portion of the device may be made approximately 0.5 mm shorter.

To avoid the risk that the radiation source may drop out of the reservoir after insertion, the walls of the reservoir are notched after introduction of the iridium-192 bar therein. This effectively locks the iridium-192 bar in place. Therefore, in a preferred embodiment according to the present invention, the inner wall of the reservoir includes a plurality of inwardly projecting bulges to keep the radiation source positioned within the reservoir. The bulges are formed at a slightly greater distance from the open end of the reservoir, than the length of the elongated portion of the plug.

In a another embodiment of the device according to the present invention, the capsule is formed integrally with the cable and constitutes a rigidly fused or melted forward portion. The capsule is provided with a bore for accommodating the radiation source, which bore is sealingly closed at its front end by welding, preferable by plasma welding. In this embodiment, the length of the rigid portion is considerably reduced, because neither a separate plug nor a plug integrally formed with the cable is required. Therefore, in this embodiment, the length of the rigid portion of the device may be a small as 0.5 to 1 mm greater than the inner length of the reservoir containing the radiation source. To manufacture such a device, the foremost portion of the cable is fused or melted, after which a bore is drilled in the rigid portion for accommodating the iridium-192 radiation source. Following insertion of the radiation source, the open front end of the bore can be sealingly closed, preferably by using the technique of plasma welding.

In a further embodiment according to the present invention, a capsule having an open end and a closed end is used. The closed end of the capsule is welded to the front end of the cable using any suitable technique, such as plasma welding. The open end of the capsule is sealed with a plug having an elongated portion for insertion into the open end of the reservoir. The plug also includes an extended portion which extends in a direction opposite from the elongated portion. The extended portion is connected to the main portion of the plug through an indented break away point. The extended portion of the plug enables a draw test to be conducted to check the strength and reliability of the weld between the capsule and cable. Once such a draw test has been satisfactorily completed, the extended portion of the plug may be broken off at the indented break away point.

Irradiation of enriched iridium-191 instead of normal iridium-191 in a nuclear reactor results in an approximately two time greater specific activity or iridium-192. Therefore, by using a radiation source produced by irradiation of enriched iridium-191, the volume of the reservoir for the radiation source can re considerably reduces, e.g. approximately by a factor of two. In this way, the length of the reservoir needed by a radiation activity of approximately 10 Curie can be reduced from approximately 4.5 mm to less than 2 mm. In other words, more than a 2 mm reduction in the length of the rigid portion of the device can be achieved.

The invention will now be described in greater detail with reference to the drawing, in which;
Figure 1 is a longitudinal sectional view of a preferred embodiment of a device according to the invention,
Figure 2 shows on an exaggerated scale a part of the device shown in Figure 1, namely the part encircled at A, and
Figure 3 shows a longitudinal sectional vies of a further preferred embodiment of a device according to the invention.

The device shown for introducing a source of radioactive radiation into the body for therapeutic application comprises a flexible cable 10 having at its rear end a solid portion 11 for including information on the radiation source and at its front end a capsule 12. The flexible cable is twisted from a large number of strands of stainless steel. The cable has a diameter of approximately 1.1 mm and can be introduced into a patients's body through a catheter having an inside diameter of at least 1.3 mm. In this manner the deeper-situated organs can be reached by means of the radiation source accommodated in the capsule for the in situ treatment of tumors. The flexible cable may have any desired length depending on the place in the body which is to be reached and on the apparatus for positioning. The solid portion 11 is connected to the cable by welding.

The radioactive material 14 consisting of a single bar of iridium-192 with a capacity of radioactivity of approximately 10 Curie is enclosed in the capsule 12. The capsule consists of a thin-walled cylindrical reservoir of stainless steel having an externally rounded-off bottom 15 which is present at the front end of the device. The open end of the reservoir facing the flexible cable is sealed by means of a plug which is formed integrally with the cable by fusing or melting. The plug includes a first portion 16 having a reduced diameter extending within the cylindrical reservoir, which keeps the radioactive material enclosed within the reservoir and tightly fits within the wall 17 of the reservoir. A second portion 18 of the plug is formed integrally with first portion 16 and bears with its outer edge or flange on the rear edge of the wall 17 of the reservoir. As is clearly shown in Figure 2, the flange and the wall of the reservoir are circumferentially welded together by a weld 19, for example, by electron beam welding or laser welding, so that a reliable and hermetic seal of the reservoir is obtained. The plug is connected to the flexible cable, by another weld 20 at the prepared end 21 of the cable, also, for example, by laser welding or electron beam welding. As noted above, the plug is formed integrally with the cable, so that the first portion 16, extending within the reservoir, forms a part of the prepared end of the cable. Stringent requirements have to be imposed upon the outside dimensions of the rigid capsule 12 in order to reach the deeper-situated organs by passing through a catheter. A cylindrical reservoir having an external length of approximately 4.5 mm, measured from the front end of the bottom 15 to the rear edge of the flange of the second portion 18 of the plug, i.e. to the end of the prepared end of the cable, and having an outside diameter of approximately 1.1 mm is optimum because such dimensions provide sufficient space for a quantity of radioactive material, in particular a single bar of iridium-192, with a radioactivity capacity of 10 Curie. Such a quantity of radioactivity is extremely suitable for the radiation treatment of tumors of internal organs, for example, lung tumors.

The wall 17 of the reservoir includes a plurality of notches 20, which appear as inwardly projecting bulges. The notches 20 act to keep the radiation source properly positioned within the reservoir, and help to avoid the risk of the iridium-192 bar dropping out of the reservoir.

Figure 3 shows a preferred embodiment of the present invention. In this embodiment a capsule 110, having an open end 112 and a closed end 114, is joined to flexible cable 100. In particular, the closed end 114 of capsule 110 is welded to cable 100, as indicated by weld 120. The capsule 110 includes a reservoir for containing a single bar 130 of iridium-192 which acts as a radiation source. The open end 112 of capsule 110 is hermetically sealed by means of a plug 140, which includes an elongated portion 142 for insertion into the open end 112 of capsule 110. The plug 140 further includes an extended portion 144, connected to the main body of the plug through an indented break point 146. The elongated portion 142 of plug 140 may be on approximately 0.5 mm in length, and still provide adequate sealing of the capsule 110. The extended portion 144 of plug 140 may be used as a grip portion during draw testing in order to assure the strength and reliability of weld 120. Following a successful draw test, the extended portion 144 may be broken off from the main body of plug 140 at break point 146. The device according to this embodiment is constructed in such a manner that the length of the rigid portion of the device may be from 0.5 to 1 mm greater than the length needed to accommodate the bar 130 of iridium-192. In particular, the rigid portion of the device according to this embodiment is approximately 4.5 mm in length. If an enriched iridium-192 source is utilized, the length of the rigid portion of the device may be reduced even further.

## Claims

1. A therapeutic device for introducing a source of radioactive radiation to a body comprising:
a flexible cable (10) which can be introduced into the body through a catheter; and
a capsule (12) for sealingly containing a source of radioactive radiation having a radiation activity of at least substantially 10 Curie, the capsule comprising a thin-walled tubular reservoir having an open end which is sealingly connected to the flexible cable so that the capsule is coaxial to the flexible cable and having a closed end (15) with a rounded-off external shape, wherein the reservoir has an outside diameter no greater than substantially 1.1 mm;
said device being characterized in that the reservoir has an external length including the sealing connection to the flexible cable of no more than substantially 4.5 mm; and that the radioactive source (14) is in the form of a single bar.

2. A device according to claim 1, wherein the open end of the reservoir is hermetically sealed to the flexible cable by means of a plug.

3. A device according to claim 2, wherein the plug is welded to the flexible cable.

4. A device according to claim 2, wherein the plug is formed integrally with the flexible cable.

5. A device according to claim 1, wherein the flexible cable is made of stainless steel.

6. A device according to claim 1, wherein the capsule is made of stainless steel.

7. A device according to claim 1, further comprising a plurality of inwardly projecting bulges (20) formed on the interior wall of said reservoir, said bulges acting to retain said radioactive source within said reservoir.

8. A device according to claim 1, wherein said capsule is formed integrally with said cable and comprises a rigidly fused or melted portion of said cable, and wherein said capsule includes a bore for accommodating said radioactive source, said bore being sealingly closed at a front end by welding.

9. A device according to claim 8, wherein said bore is sealingly closed by plasma welding.

10. A device according to claim 1, wherein said radioactive source comprises a single bar of iridium-192.

11. A device according to claim 10, wherein said iridium-192 is formed by irradiation of enriched iridium-191.

12. A device according to claim 1, wherein the closed end (114) of the reservoir (110) is welded to the flexible cable (100) and wherein the open end (112) of the reservoir is hermetically sealed by means of a plug (140), said plug including a main body portion, an elongated portion (142) for insertion into said open end of said capsule, and an extended portion (144), extending in a direction opposite of said elongated portion and being connected to said main body portion through an indented break point (146).

## Patentansprüche

1. Therapeutische Vorrichtung zur Einführung einer Radioaktiven Strahlungsquelle in einen Körper, wobei die Vorrichtung aufweist:
ein flexibles Kabel (10) das in den Körper durch einen Katheter eingeführt werden kann; und
eine Kapsel (12), die eine radioaktive Strahlungsquelle mit einer Strahlungsaktivität von im wesentlichen mindestens 10 Curie dicht aufnimmt und die einen dünnwandigen röhrenförmigen Behälter aufweist, der ein offenes Ende hat, welches dicht mit dem flexiblen Kabel so verbunden ist, daß die Kapsel koaxial zum flexiblen Kabel liegt und der ein geschlossenes Ende (15) mit abgerundeter Außenform hat, wobei der Behälter einen Außendurchmesser nicht größer als im wesentlichen 1,1 mm hat;
wobei die Vorrichtung dadurch gekennzeichnet ist, daß der Behälter eine dichte Verbindung mit dem flexiblen Kabel einschließende Außenlänge von nicht mehr als im wesentlichen 4,5 mm hat; und daß die radioaktive Strahlungsquelle (14) die Form eines einzelnen Stabs hat.

2. Vorrichtung nach Anspruch 1, bei der das offene Ende des Behälters hermetisch dicht mit dem flexiblen Kabel mittels eines Stopfens verbunden ist.

3. Vorrichtung nach Anspruch 2, bei der der Stopfen am flexiblen Kabel angeschweißt ist.

4. Vorrichtung nach Anspruch 2, bei der der Stopfen einstückig mit dem flexiblen Kabel ausgebildet ist.

5. Vorrichtung nach Anspruch 1, bei der das flexible Kabel aus rostfreiem Stahl hergestellt ist.

6. Vorrichtung nach Anspruch 1, bei der die Kapsel aus rostfreiem Stahl hergestellt ist.

7. Vorrichtung nach Anspruch 1, welche weiterhin eine Vielzahl nach innen ragender Wulste (20) aufweist, welche auf der Innenwand des Behälters ausgebilded sind, wobei diese Wulste als Halterung für die radioaktive Strahlungsquelle innerhalb des Behälters wirken.

8. Vorrichtung nach Anspruch 1, bei der die Kapsel einstückig mit dem Kabel ausgebildet ist und einen starr geschmolzenen oder geschweißten Abschnitt des Kabels aufweist und bei der die Kapsel eine Bohrung zur Aufnahme der radioaktiven Quelle enthält, welche Bohrung an ihrer Stirnseite durch Verschweißung dicht verschlossen ist.

9. Vorrichtung nach Anspruch 8, bei der diese Bohrung durch Plasmaverschweißung dicht verschlossen ist.

10. Vorrichtung nach Anspruch 1, bei der die radioaktive Strahlungsquelle einen einzelnen Stab aus Iridium-192 aufweist.

11. Vorrichtung nach Anspruch 10, bei der das Iridium-192 durch Bestrahlung von angereichtertem Iridium-191 gebildet ist.

12. Vorrichtung nach Anspruch 1, bei der das geschlossene Ende (114) des Behälters (110) mit dem flexiblen Kabel (100) verschweißt ist, und bei der das offene Ende (112) des Behälters mit einem Stopfen hermetisch abgedichtet ist, wobei dieser Stopfen einen Hauptkörperabschnitt und einen verlängerten Abschnitt (142) zum Einfügen in das offene Ende der Kapsel sowie einen vorstehenden Abschnitt (144) aufweist, der sich in der dem verlängerten Abschnitt entgegengesetzten Richtung erstreckt und der mit dem Hauptkörper durch eine eingekerbte Bruchstelle (146) verbunden ist.

## Revendications

1. Un dispositif thérapeutique pour l'introduction d'une source de radiation radioactive dans un corps, comprenant:
un câble souple (10) qui peut être introduit dans le corps par l'intermédiaire d'un cathéter; et
une capsule (12) pour recevoir de façon étanche une source de radiation radioactive ayant une radioactivité, sensiblement, d'au moins 10 curies, la capsule comprenant un réservoir tubulaire à paroi mince ayant une extrémité ouverte qui est reliée de façon étanche au câble souple, de manière que la capsule soit coaxiale au câble souple et comprenant une extrémité fermée (15) de forme extérieure arrondie, le réservoir ayant un diamètre extérieur ne dépassant pas, sensiblement, 1,1 mm;
ledit dispositif étant caractérisé en ce que le réservoir a une longueur extérieure comprenant le raccord étanche avec le câble souple, ne dépassant pas sensiblement 4,5 mm; et que la source radioactive (14) est en forme d'un barreau unique.

2. Un dispositif selon la revendication 1, dans lequel l'extrémité ouverte du réservoir est scellée hermétiquement sur le câble souple au moyen d'un bouchon.

3. Un dispositif selon la revendication 2, dans lequel le bouchon est soudé sur le câble souple.

4. Un dispositif selon la revendication 2, dans lequel le bouchon est réalisé monobloc avec le câble souple.

5. Un dispositif selon la revendication 1, dans lequel le câble souple est réalisé en acier inoxydable.

6. Un dispositif selon la revendication 1, dans lequel la capsule est réalisée en acier inoxydable.

7. Un dispositif selon la revendication 1, comprenant en outre une pluralité de dentelures ou rainures en saillie vers l'intérieur (20), ménagées dans la paroi intérieure dudit réservoir, lesdites dentelures agissant pour retenir la source radioactive à l'intérieur dudit réservoir.

8. Un dispositif selon la revendication 1, dans lequel ladite capsule est réalisée monobloc avec ledit câble et comprend une partie fondue ou soudée rigidement dudit câble, et dans lequel ladite capsule comprend un orifice pour recevoir ladite source radioactive, ledit orifice étant obturé de façon étanche à l'extrémité avant par soudage.

9. Un dispositif selon la revendication 8, dans lequel ledit orifice est fermé de façon étanche par soudage au plasma.

10. Un dispositif selon la revendication 1, dans lequel ladite source radioactive comprend un barreau unique en iridium-192.

11. Un dispositif selon la revendication 10, dans lequel ledit iridium-192 est formé par irradiation d'iridium-191 enrichi.

12. Un dispositif selon la revendication 1, dans lequel l'extrémité fermée (114) du réservoir (110) est soudée sur le câble souple (100) et dans lequel l'extrémité ouverte (112) du réservoir est fermée hermétiquement au moyen d'un bouchon (140), ledit bouchon comprenant une partie de corps principal, une partie oblongue (142) à introduire dans ladite extrémité ouverte de ladite capsule, et une partie en extension ou prolongement (144), s'étendant dans une direction opposée de ladite partie oblongue et étant reliée à ladite partie de corps principal par une zone de fracture à amorce de rupture (146).
